# EUROPÄISCHE PATENTSCHRIFT

(11) **EP 0 673 656 B1**
(45) Veröffentlichungstag und Bekanntmachung des Hinweises auf die Patenterteilung: **20.08.1997**
(21) Anmeldenummer: 95104200.1
(22) Anmeldetag: 22.03.1995
(51) Int. Cl.: A61L 9/20, A61L 9/16, A61L 9/18, A61L 9/22

(54) **Verfahren zum Reinigen von Luft und Vorrichtung zur Durchführung des Verfahrens**
Air purifying process and device
Procédé et appareil pour la purification de l'air

(30) Priorität: 25.03.1994 CH 900/94
(43) Veröffentlichungstag der Anmeldung: 27.09.1995
(73) Patentinhaber: Perentron Engineering Limited, Rathfarnham, Dublin 16 (IE)
(72) Erfinder:
(74) Vertreter: Büchel, Kurt F., Dr.

(56) Entgegenhaltungen:
- DE-A- 2 836 708
- DE-A- 3 735 251
- FR-A- 1 347 772
- FR-A- 2 448 930
- GB-A- 2 112 298
- GB-A- 2 212 370

## Beschreibung

Verschiedentlich ist die Luft, und insbesondere die Luft in Arbeitsräumen, vornehmlich in Betrieben der Nahrungsmittelbranche, hohen Schadstoffbelastungen ausgesetzt, die zu Erkrankungen führen können. Besonders schwierige Verhältnisse finden sich in Bäckereien, wo verschiedene Allergene, aber auch Parasiten und Mikrolebewesen zu Erkrankungen der Atemwege führen, die allgemein mit dem Begriff "Bäckerasthma" bezeichnet werden, tatsächlich aber nicht nur auf Allergene, sondern auch auf das Einwirken der Schädlinge auf die durch erstere bereits gereizten und entzündeten Bronchien zurückzuführen sind. Es ist verständlich, dass das gehäufte Auftreten der Schädlinge verschiedenster Art auf die für sie besonders günstigen Bedingungen, nämlich das durch Mehl etc. für sie vorhandene Nahrungsangebot sowie die für eine Vermehrung relativ günstigen Temperaturen zurückzuführen ist. Es versteht sich daher, dass die vorliegende Erfindung zwar bevorzugt in Bäckereien Anwendung findet, dass aber die zu lösenden Probleme überall dort auftreten, wo eine ähnliche Kombination von verschiedenen Schadstoffen und Bedingungen zu finden ist, was besonders in Betrieben der Nahrungsmittelbranche der Fall sein wird.

Wenn hier von Schädlingen und Schadstoffen die Rede ist, so muss man - und dies ist für eine genaue Analyse des Problems unerlässlich - wissen, um welche Arten von Schädlingen und Schadstoffen es sich handelt.

Zunächst ist hier von einer gewissen Staubbelastung die Rede, wozu erschwerend hinzutritt, dass dieser Staub auch noch den Nährboden für pflanzliche und tierische Kleinlebewesen abgibt, wenn es sich - wie in Bäckereien - um Mehl handelt. Die Staubbelastung führt naturgemäss aber auch zu einem weiteren Problem, nämlich dem möglicher Staubexplosionen.

Medizinische Untersuchungen zeigten, dass es vor allem Bakterien sind, die auf Nahrungsmittelstäuben einen guten Nährboden finden und sich dann am allergenen Geschehen beteiligen. Untersuchungen weisen auf den Einfluss von Toxinen ebenso hin, wie auf die Auslösung von Superantigenwirkungen.

Eine andere Gruppe vielfach anzutreffender Kleinlebewesen sind Pilze verschiedener Art, die naturgemäss an den sich ergebenden Erkrankungen nicht unbeteiligt sind. Vor allem ist es hier die von vielen Pilzen ausgeschiedene Amylase, die ein potentes Allergen darstellt.

Daneben gibt es aber auch grössere Parasiten, wie auch Käfer, z.B. Reismehl- und Kornkäfer, Milben und Motten, wobei diese Parasiten wiederum nicht nur ein hygienisches Problem darstellen, sondern auch - teilweise zusammen mit den Pilzen - ein Problem für die Haltbarkeit von Luftreinigungseinrichtungen bilden. Insbesondere Dichtungen sind leicht dem Käferfrass ausgesetzt, so dass an das Material erhöhte Anforderungen gestellt werden müssen.

Im Falle der Befeuerung, wie sie in Bäckereien notwendig ist, treten dann verschiedene gasförmige Schadstoffe, wie Schwefeldioxyd oder Kohlenmonoxyd und -dioxyd hinzu. Ausserdem sind verschiedentlich auch noch polycyclische aromatische Kohlenwasserstoffe, Acroleine und Aldehyde zu nennen.

Nun ist für das Problem einer Bakterien enthaltenden Raumluft bereits verschiedtlich Abhilfe vorgeschlagen worden. So beschreibt die DE-A-20 63 762 eine Vorrichtung und ein Verfahren nach den Oberbegriffen der Ansprüche 6 bzw. 1. Einerseits ist diese Vorrichtung nicht für übermässig mit Bakterien und Staub sowie anderen Schadstoffen beladene Luft gedacht, sondern nur für normale Luft, deren Bakteriengehalt für Sterilräume, wie Operationsräume, ausgefiltert werden soll. Insoferne sind also dort die Gegebenheiten erleichtert, wenn auch der Gedanke, die Mikroben möglichst schon am oder vor dem Filter zu vernichten, in die richtige Richtung geht.

Allerdings ist diese bekannte Lösung für die Zwecke der vorliegenden Erfindung, nämlich eine Reinigung der Luft in extrem mit Schadstoffen und Schädlingen beladenen Räumen durchzuführen, kaum brauchbar. Der Grund hiefür liegt einfach darin, dass - wie oben erwähnt - verschiedene Kleinlebewesen, wie Käfer, einerseits mit einer UV-Bestrahlung nicht umzubringen sind, anderseits dadurch ihr zerstörerisches Werk an den Filteranlagen ungehindert weiter fortzusetzen vermögen. Der Effekt ist dann, dass selbst die angestrebte Wirkung, nämlich Bakterien und Staub auszufiltern, nicht mehr erreicht werden kann.

Aus der DE-A-36 24 803 ist auch eine andere Kombination von Massnahmen bekannt geworden, die teilweise auch für die vorliegenden Zwecke Anwendung finden können. Aber auch die Behandlung mit Ozon bzw. eine Ionisierung reicht nicht aus, um dem Befall durch die gefährlichen Parasiten Einhalt zu gebieten.

Der Erfindung liegt daher die Aufgabe zugrunde, für übermässig mit Bakterien und Staub sowie anderen Schadstoffen beladene Luft, wie man sie in Betrieben der Nahrungsmittelerzeugung, insbesondere Bäckereien, vorfindet, ein Verfahren und eine Vorrichtung vorzuschlagen, die den besonderen, erschwerten Verhältnissen gerecht wird. Erfindungsgemäss gelingt die Lösung dieser Aufgabe durch die im Kennzeichen des Anspruches 1 beschriebenen Merkmale. Vorteilhafte Weiterbildungen der Erfindung sind in den abhängigen Ansprüchen beschrieben.

Die erfindungsgemässe Kombination ergibt in verschiedener Hinsicht eine synergetische Wirkung. Denn die vorgeschlagene Beschallung mit Ultraschall tötet nicht nur grössere Parasiten, sondern teilweise auch Mikroben, so dass die Probleme in der zweiten Stufe leichter in den Griff zu bringen sind. Vor allem ist nun eine längere Lebensdauer von Filtern und Dichtungen gesichert und damit eine verlässliche Funktion derselben. Andererseits ist die Beschallung zwar für erwachsene Parasiten dieser Art letal, nicht aber etwa für manche Eier etc.. Diese aber werden durch die nachfolgende zweite Stufe mit Sicherheit vernichtet, so dass auch hier ein Zusammenwirken der Massnahmen stattfindet.

Wie schon erwähnt, wird sich die an sich bekannte Ionisierung für viele Anwendungsfälle als zweckmässig erweisen. Wie aus der späteren Beschreibung noch ersichtlich wird, ist eine Verstellbarkeit der Leistung einer Ionisiereinrichtung besonders vorteilhaft.

Im Hinblick auf die Komplexität des anvisierten Problems ist es auf Grund der obigen Erläuterungen wohl verständlich, warum die Massnahmen nach Anspruch 3 für eine verbesserte Lösung der oben definierten Aufgabe günstig sind.

In erstaunlicher Weise hat sich herausgestellt, dass eine Kühlung der Raumluft, und dies ist in einer Bäckerei prinzipiell nichts Unangenehmes, eine erhebliche Erleichterung bringt, weil dadurch die Lebensbedingungen für Schädlinge empfindlich verschlechtert werden. Wenn man sich vor Augen hält, dass Kleinlebewesen auf Temperaturveränderungen sehr stark ansprechen, ist es wohl verständlich, dass bereits eine Absenkung der Raumtemperatur um wenige Grade einen deutlichen Effekt haben kann.

Zur Durchführung des erfindungsgemässen Verfahrens wird eine Vorrichtung mit den Merkmalen des Anspruches 6 vorgeschlagen.

Weitere Einzelheiten der Erfindung ergeben sich an Hand der nachfolgenden Beschreibung von in der Zeichnung schematisch dargestellten Ausführungsbeispielen. Es zeigen:
- Fig. 1: ein Blockschema einer erfindungsgemäss ausgebildeten Anlage bzw. Vorrichtung, an Hand dessen das erfindungsgemässe Verfahren in einer bevorzugten Ausführungsform erläutert wird;
- Fig. 2: einen Rohrquerschnitt mit einem optischen Messwertgeber zur Messung der Staubkonzentration;
- Fig. 3: den Aufbau einer einen Ultraschall- und/oder Hochfrequenzgeber, ein Grobsieb und eine zugeordnete Vibrationseinrichtung umfassenden Einheit in einer schematischen Perspektivansicht ohne die Rohrwandung; und
- Fig. 4: eine Perspektivansicht einer UV-Bestrahlungseinheit bei weggelassener Rohrwand.

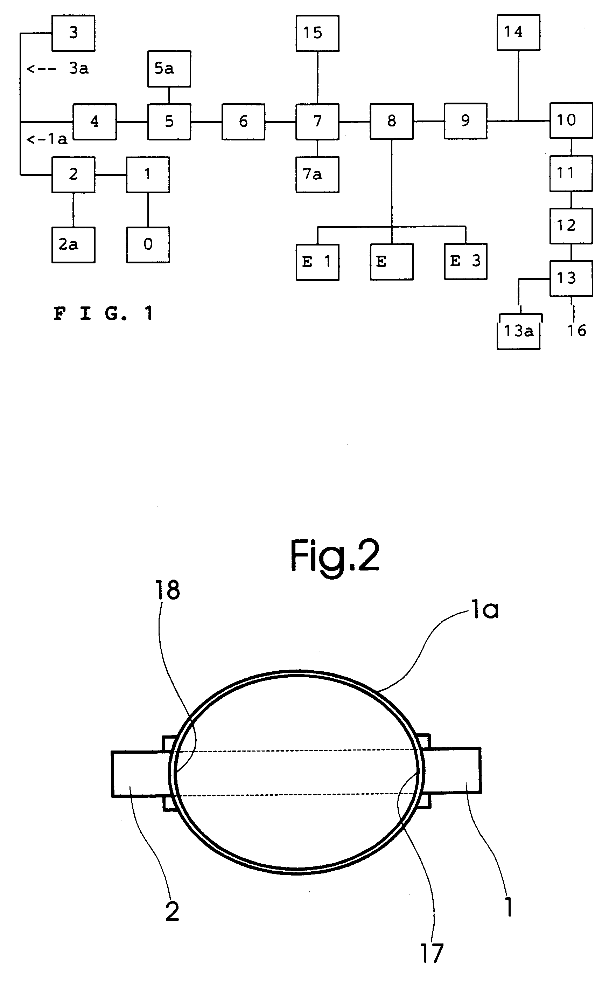

Eine erfindungsgemässe, an Hand der Fig. 1 schematisch dargestellte Vorrichtung geht von einer Ansaugung 0 für die zu reinigende Abluft bzw. Raumluft aus. Ein anschliessendes und allgemein mit 1 bezeichnetes Rohrsystem hat im allgemeinen aus korrosionsbeständigem Material zu sein, da die bereits erwähnten Schadstoffe und Ausscheidungen der Kleinlebewesen es andernfalls einer raschen Alterung aussetzen und wiederum zur Heimstätte für derlei Lebewesen machen würden. Es versteht sich, dass hier glatte, leicht zu reinigende und allenfalls zu desinfizierende Rohre von Bedeutung sind, wobei gegebenenfalls Reinigungs- und/oder Desinfizierungseinrichtungen (z.B. Sprinkler) eingebaut sein können. Für eine mechanische Reinigung kann gewünschtenfalls eine Molch- oder eine ähnliche Anlage zugeordnet sein. Bevorzugt sind Rohre aus rostfreiem Stahl, die auch wegen einer allfälligen Staubexplosionsgefahr den Vorteil der elektrischen Leitfähigkeit besitzen. Dabei ist die Leitung zweckmässig aus einzelnen, über Verbindungseinrichtungen, z.B. auch Schnellkupplungen, miteinander verbindbaren Modulen aufgebaut, so dass die Zugänglichkeit für allfällige Reinigungsarbeiten erleichtert ist. Andererseits müssen die einzelnen Rohrschüsse miteinander dicht verbunden sein und weisen daher entsprechende, in der einschlägigen Technik an sich bekannte Dichtungen auf.

Zweckmässig gleich anschliessend an den Eingang 1 eines Rohrleitungssystems 1a ist vorteilhaft eine Messeinrichtung 2 vorgesehen, die Teil einer Steuer- bzw. Regeleinrichtung 2a sein kann. Auf letztere wird später noch näher eingegangen. Messgeräte zur Messung der Staubkonzentration sind in verschiedener Ausführung bekannt, so dass diese an sich hier nicht beschrieben zu werden brauchen. Es sei lediglich erwähnt, dass für die vorliegenden Zwecke ein optisches Messsystem als genau genug befunden wurde, und ein solches, hier besonders zweckmässiges, wird später an Hand der Fig. 2 beschrieben. Die Signale des Messwertgebers 2 werden einer Reguliereinrichtung 2a zugeführt, die als blosse Steuer- oder als Regeleinrichtung ausgebildet sein kann. Diese Einrichtung 2a mag dazu dienen, einzelne Betriebsparameter, wie die Intensität der die Schädlinge bekämpfenden Massnahmen, z.B. zeitabhängig (etwa über den Tagesverlauf) und/oder in Abhängigkeit von weiteren Sensoren innerhalb der Einrichtung 2, einzustellen. Beispielsweise kann die Einrichtung auch einen Temperatursensor umfassen, wobei eine höhere Temperatur im Bereiche optimaler Lebensbedingungen der Schädlinge zu einer verstärkten Bekämpfung derselben, sei es durch Verstärkung der Intensität der Beschallung bzw. der Ionisierung, sei es durch vermehrte Versprühung von Desinfektionsmitteln, führen kann. Es kann sogar ein Temperaturprofil derart in der Einrichtung 2a abgespeichert sein, dass in einem Temperaturbereich die eine Massnahme verstärkt wird, in einem anderen Temperaturbereich eine andere. Ebenso könnte ein Wochenprofil (evt. auch ein Jahresprofil zur Berücksichtigung der Jahreszeiten) abgespeichert sein, da erfahrungsgemäss die Verhältnisse nach einem Ruhetag andere sind als bei stets laufendem Betrieb. Da all diese Faktoren in der Beurteilung eher unscharf zu bewerten sind, eignet sich hier eine Fuzzy-Logik am besten, doch könnte auch ein Expertensystem mit dem Wissen, wann und zu welchen Bedingungen welche Parasiten besonders zu bekämpfen sind, angewandt werden. Dies wird im allgemeinen bedingen, dass die Einrichtung 2a als Prozessorsystem, z.B. mit einem Mikroprozessor, aufgebaut ist.

In das Rohrleitungssystem 1a mündet gegebenenfalls mindestens eine Zweigleitung 3a ein, die von einer Lokalabsaugung 3, z.B. mit einem Schlauchsystem (z.B. als sog. Polypsystem auch mit mehreren Leitungen bzw. Schläuchen), an einem von Schadstoffen besonders betroffenen Orte her geführt ist. Somit wird die Luft sowohl von der allgemeinen Ansaugstelle 1 als auch von der mindestens einen Lokalabsaugung zweckmässig zusammengefaßt, um z.B. einem Ultraschallgeber 4 zur Beschallung zugeführt zu werden. Da die Lokalabsaugung 3, z.B. eine im Bereiche eines Backtisches fix eingebaute Lokalabsaugung, gegebenenfalls nicht ständig betrieben werden muss, ist es vorteilhaft, wenn sie gewünschtenfalls ein eigenes Gebläse besitzt, und/oder z.B. über Schaltkontakte eine Anpassung der jeweiligen Gebläseleistung erfolgt.

Es wurde bereits erwähnt, dass die Leistung der einzelnen Bekämpfungsmittel für die Parasiten veränderbar sein kann, und dass hiefür zweckmässig eine Automatik mit mindestens einem Messwertgeber 2 vorgesehen ist. In diesem Sinne ist es zu verstehen, dass gegebenenfalls auch die vom Ultraschallgeber abgegebene Energie veränderlich sein kann, obwohl es zur sicheren Abtötung von Schädlingen gegebenenfalls nötig sein wird, eine untere Grenze vorzusehen. Im allgemeinen wird hier aber eine Festeinstellung der Energie durchaus genügen. Durch den Ultraschallgeber werden Schwingungen an die Parasiten übertragen, die sich an ihnen, gegebenenfalls in Form einer örtlichen Temperaturerhöhung, über das ihnen zuträgliche Mass hinaus auswirken können. Daher versteht es sich, dass unter Umständen die Anordnung eines Mikrowellensenders bzw. eines Hochfrequenzfeldes an Stelle des Ultraschallgebers 4 eine alternative bzw. äquivalente Massnahme sein kann. Es wurde aber bereits gesagt, dass auch die Probleme des Explosionsschutzes im allgemeinen zu berücksichtigen sein werden, und hier ist ein Ultraschallgeber 4 von besonderem Vorteil.

Im Hinblick auf die Erleichterung der anschliessend getroffenen und noch zu beschreibenden Massnahmen ist es zweckmässig, im Anschlusse an die in einer ersten Stufe vorgängige Beschallung bei 4 die zweite Behandlungsstufe mit einer ersten Filtrierung, insbesondere zunächst einmal durch ein Grobsieb 5 beginnen zu lassen, das eine Maschenweite von 0.1 bis 0.4 mm, insbesondere von etwa 0.25 bis 0.3 mm besitzt, so dass eine etwaige Grobfiltrierung der oben genannten Art erst später erfolgt. Die durch die Beschallung abgetöteten, grösseren Schädlinge werden dabei vorher ausgefiltert und belasten daher dann die weitere Behandlung nicht.

Bei vertikaler Anordnung des Siebes 5 ist dieses gegebenenfalls selbstreinigend, weil die abgefangenen Schädlinge vor dem Sieb liegenbleiben und es nicht verstopfen. Es kann aber zusätzlich von Vorteil sein, wenn das Sieb 5 vibriert wird, um hängengebliebene Teilchen abzuschütteln. Zu diesem Zweck kann am Sieb 5 ein Vibrator 5a, z.B. mit einer Frequenz unter 100 Hz, z.B. 50 Hz, befestigt werden.

Die Grobfiltrierung erfolgt am günstigsten mit einem Filter, welches ein Korn von 1 bis 1.5 µm und grösser gerade noch abfängt. Filter mit einer Porengrösse von 1.2 µm haben sich in der Praxis bewährt. Dies bedeutet, dass feinere Stäube zunächst durchgelassen und erst bei der anschliessenden Feinfiltrierung ausgeschieden werden. Dies hat auch seinen besonderen Grund. Dadurch nämlich, dass die Filtrierung in wenigstens zwei Stufen erfolgt, werden die Abreinigungszeiten erhöht, gleichzeitig aber die Leistung und die Zuverlässigkeit des Systems vergrössert.

Die Anordnung eines Siebes 5 in Form eines Metallsiebes hat den zusätzlichen Vorteil einer Ableitung allfälliger statischer Aufladungen, so dass dadurch die Explosionsgefahr (nach Art des Prinzipes einer Grubenlampe) vermindert wird. Aus diesem Grunde mag es zweckmässig sein, auch an einem späteren Ort des Rohrleitungssystems 1a, insbesondere am Ausgang der in Fig. 1 gezeigten Reinigungsanlage, ein solches Sieb aus Metalldraht vorzusehen, um die Übertragung allfälliger Verpuffungen auf das Rohrsystem 1a zu vermeiden.

Nötigenfalls kann das Sieb 5 mit einem in den Luftweg einschiebbaren Rahmen, jedenfalls leicht auswechselbar, ausgebildet sein, um bei Störungen am Sieb die Anlage möglichst unterbrechungsfrei weiterhin betreiben zu können. Analoges gilt für die später noch zu beschreibende Filteranordnung.

Um die Belastung des Personals durch Lärmeinwirkung möglichst gering zu halten, ist im Zuge des Rohrleitungssystemes 1a mindestens ein Schalldämpfer 6 vorgesehen. Dieser Schalldämpfer 6 wird zweckmässig am dargestellten Ort, d.h. unmittelbar hinter der Grobsiebung bei 5 angebracht, doch können, je nach den besonderen Verhältnissen zusätzlich oder alternativ, auch andere Anbringungsorte zweckmässig sein. Beispielsweise mag es erforderlich sein, vor und/oder nach einem der Ventilatoren (falls mehrere vorgesehen sind) jeweils einen Schalldämpfer anzubringen. Die Ausbildung des Schalldämpfers 6 erfolgt entsprechend der bekannten einschlägigen Technik. Dem Grobsieb kann auch ein Feinsieb mit einer Maschenweite von z.B. 10 bis 30 µm nachgeschaltet sein.

Bei 7 ist eine Filtrierstufe gezeigt, die in sich wiederum ein- oder mehrstufig ausgebildet sein kann. Bewährt hat sich eine Unterteilung in ein Grobfilter, wie es oben bereits an Hand des Siebes 5 als eine Möglichkeit mit einer Korntrenngrenze von 1 bis 1.5 µm, insbesondere von etwa 1.2 µm, beschrieben wurde, und anschliessend ein Kaskadenkammerfilter, das die feineren Stäube entfernt. Durch diese Arbeitsteilung haben die Filter bis zur jeweils nächsten Abreinigung eine längere Standzeit. Die Funktionstüchtigkeit der Filter wird zweckmässig in der für Filter üblichen Weise mittels Druckdifferenzmessung überwacht und zu gegebener Zeit, d.h. bei zu hohem Differenzdruck zwischen Staubseite und Reinseite, ein Alarm oder eine der üblichen Abreinigungsmassnahmen ausgelöst, wie sie dem Fachmann auf diesem Gebiete bekannt sind. Zur Messung des Differenzdruckes ist mit dem Filtersystem 7 eine Messeinrichtung 15 verbunden.

Bewährt haben sich Taschenfilter, die allerdings wegen der anvisierten erschwerten Bedingungen aus möglichst beständigem Material, wie Glasfasern, z.B. mit einer Beschichtung, wie einer PVC-Beschichtung, aufzubauen sind. Hier ist es, wie Fig. 1 deutlich zeigt, von Vorteil, wenn an das Filtersystem 7 gleich auch eine Sprüheinrichtung 7a für die Sprühvernebelung eines Desinfektionsmittels angeschlossen ist. Dies hat einen doppelten Vorteil. Denn einerseits erleichtert die versprühte Flüssigkeit die Abreinigung, indem sie die an der Staubseite angesammelten Staubteile agglomeriert. Andererseits wird so gesichert, dass die feinen Tröpfchen nicht verloren gehen, sondern an der Oberfläche des Filters haften bleiben und dort eine relativ lange Wirkungsdauer besitzen. Die Sprüheinrichtung umfasst selbstverständlich ein entsprechendes Reservoir für wenigstens ein Desinfektionsmittel, wie eine quaternäre Ammoniumverbindung, doch kann es, besonders bei einer mehrstufigen Filteranlage 7, zweckmässig sein, jedem Filter eine andere Desinfektionsflüssigkeit zuzuordnen, um so gegen ein breites Spektrum von Schädlingen gewappnet zu sein. Ebenso wäre es aber möglich, einem einzigen Filter mehrere Desinfektionsdüsen, allenfalls mit Anschluss an verschiedene Reservoirs, zuzuordnen. Im letzteren Falle könnten die Sprühdüsen zur Abgabe eines Aerosols bei einer axialsymmetrischen Filterausbildung, wie einem Schlauchfilter, um dieses herum angeordnet sein, oder es ist ein Bewegungsantrieb für die Sprühdüsen vorgesehen, wie dies im Stand der Technik bereits vorgeschlagen wurde, um eine gleichmässige Besprühung des Filters über dessen Fläche zu erreichen.

Auch die Besprühung mit Kontaktgiften ist denkbar, und es mag zweckmässig sein, wenn die Abgabe des Desinfektionsmittels in seiner Menge über die Zeit von der Prozessoreinrichtung 2a gesteuert wird. Hier ist an sich eine Analogsteuerung denkbar, wobei das Mittel dauernd abgegeben, die Menge aber, z.B. über ein Proportionalventil, verändert wird, doch ist es bevorzugt, wenn eine Digitalsteuerung vorgesehen ist, so dass die Besprühung nur in Zeitabständen eingeschaltet wird. Dies hat seinen Grund darin, dass die nötigen Mengen relativ gering sind und ihre Wirkung über eine verhältnismässig lange Zeit entfalten, so dass eine analoge Steuerung zu Ungenauigkeiten und zu ineffizientem Betrieb führen könnte.

Die beschriebene Filtrieranordnung ist aber in weitem Masse den jeweiligen Verhältnissen und Bedürfnissen anpassbar. Denn schon an Hand der Beschreibung der Stufe 5 zeigte es sich, dass die Filtrierung zweckmässig auch durch ein Sieb ausgeführt werden kann. Ebenso wäre es aber auch denkbar, in der Stufe 7 einem Staubfilter (im engeren Sinne) einen anderen Grobabscheider, wie einen Sichter, z.B. aber auch einen Zyklon, vorzuschalten. Insofern soll im Rahmen der vorliegenden Beschreibung der Begriff "Filtrierung" im weitesten Sinne einer Abscheidung verstanden werden, denn letztlich kommt es ja nur darauf an, die gewünschten Korngrössen jeweils aus dem Luftstrom herauszubekommen. Statt der Desinfizierung bzw. zusätzlich zu dieser wäre es aber auch denkbar, andere Sprühmittel in die zu reinigende Luft einzusprühen, wie Duft- und Aromastoffe zur Luftverbesserung, Antiallergentien etc.

Im Anschluss an die Filtrierstufe 7 wird im allgemeinen ein Gebläse 8, z.B. ein Sauggebläse, vorgesehen sein, dem vorteilhaft ein Schalldämpfer 9 zugeordnet, z.B. nachgeschaltet, ist. Der Schalldämpfer 9 (oder die Gesamtheit aller Schalldämpfer, wenn mehrere vorhanden sind), ist so ausgelegt, dass ein Schallpegel von maximal 60 dBA, vorzugsweise maximal 55 dBA erreicht wird. Dies dient nicht zuletzt dazu, die Geräuscherzeugung der Anlage auf einem derart niedrigen Niveau zu halten, dass sie nicht etwa zur Schonung der Ohren des Personals öfter einmal abgeschaltet wird, sondern ein durchgehender Betrieb aufrechterhalten werden kann. Bewährt hat sich ein Kulissenschalldämpfer, wobei die Strömungsgeschwindigkeit im Spalt zwischen den Kulissen bei Versuchen zwischen 9 und 15 m/sec, vorzugsweise 10 bis 12 m/sec, z.B. 11 m/sec, betrug.

Der Ventilator 8 ist, wie andere Stufen der gezeigten Anlage zweckmässig auch, in seiner Leistung veränderbar. Dazu sind beispielsweise Sensoren E1 bis E3 vorgesehen, die gegebenenfalls mit dem Prozessor 2a (in nicht dargestellter Weise) derart verbunden sind, dass etwa bei sich verschlechternden Bedingungen, wie höheren Staubkonzentrationen, die Leistung des Gebläses 8 erhöht wird. Dies ist eine weitere Massnahme, um den Betriebspunkt einerseits möglichst optimal zu halten, die damit meist einhergehende Geräuschentwicklung aber zeitlich zu begrenzen. Dabei können die Sensoren E1-E3 gegebenenfalls einfache Kontakte bzw. Schalter sein, die etwa auf das Öffnen der Backofentüre oder eines anderen Staub emittierenden oder die Bedingungen verschlechternden Organes ansprechen. Ferner wäre denkbar, dass die Sensoren E1-E3 mindestens einen manuell bzw. willkürlich einstellbaren Steller umfassen.

Im Anschluss an die Filtriereinrichtung 7 kann innerhalb des Rohrleitungssystems 1a mindestens noch eine weitere Keimtötungsstufe 10 bzw. 11 vorgesehen sein. Es ist wichtig, bei der Auswahl dieser Stufen darauf zu achten, dass nicht durch sie eine andere Verschlechterung der Atemluft, etwa durch die Erzeugung schädlicher Mengen an Ozon, hervorgerufen wird. Günstig ist die Anordnung einer UV-Bestrahlungsstufe 10 (evt. mit zuschaltbaren Lampen zur Leistungsregelung) bzw. eines Ionisators 11, dessen Leistung leicht regelbar gemacht werden kann. Hier ist die schon erwähnte Steuerung durch den Prozessor 2a zur Leistungsregulierung der Stufe 11 von besonderem Vorteil. Wie schon erwähnt, kann dabei die Steuerung in zeitlicher Abhängigkeit und/oder in Funktion des Ansprechens eines der Sensoren 2 bzw. E1-E3 erfolgen. Indirekt wäre eine Steuerung durch die Sensoren E1-E3 auch derart möglich, dass die Leistungsaufnahme des Gebläses 8 überwacht und danach die Leistung der UV-Bestrahlungsstufe 10, insbesondere aber des Ionisators 11, geregelt wird.

Zusätzlich oder alternativ zur Sprüheinrichtung kann innerhalb des Rohrleitungssystems 1a auch noch eine weitere Sprüheinrichtung 12 vorgesehen sein, und für diese gilt praktisch alles, was oben bezüglich der Sprüheinrichtung 7a gesagt wurde. Die Sprüheinrichtung 12, da ihr keine Filterfläche zugeordnet ist, wird das Sprühgut zweckmässig besonders fein vernebeln, damit eine gute Verteilung in der durch die Rohrleitung 1a strömenden Luft erzielt wird. Sprühdüsen bzw. Sprinkler mit Ultraschallzerstäubung kommen hier besonders in Frage. Es sei aber erwähnt, dass gegebenenfalls die Wirkungen dieser Komponenten miteinander kombiniert werden können, indem die Sprüheinrichtung 12 im Bereich der Ultraschalleinrichtung 4 angeordnet wird und der Schall des Ultraschallgenerators sowohl auf die Parasiten geleitet, als auch zur Zerstäubung ausgenützt wird. Auch mag die Arbeitsweise zwischen den Einrichtungen 7a und 12 derart geteilt sein, dass über die Sprüheinrichtung 7a Kontaktgifte für die Parasiten, über die Sprüheinrichtung 12 jedoch andere, flüssige Desinfektionsmittel versprüht werden.

Im Prinzip ist im Anschluss an die Stufe 12 die Reinigung beendet, so dass die Luft an eine Klimaanlage 13 zur Einführung in die jeweiligen Räumlichkeiten bzw. an einen Ausgang 16 abgegeben werden kann. Es mag hier allerdings eine weitere Steuerungsmöglichkeit (zweckmässig über den Prozessor 2a) mit Hilfe einer Einstellklappe od. einem ähnlichen Ventilorgan 14 wünschenswert sein, indem über dieses Organ die Menge an Umluft bzw. ihr Verhältnis zu zugeführter Frischluft einstellbar ist. Besonders wichtig mag in diesem Zusammenhang erscheinen, dass im Rahmen der Klimaanlage 13 gegebenenfalls auch eine Kühlanlage für die zu reinigende Luft eingebaut sein kann, die zweckmässig so dimensioniert ist, dass damit vor der Rückführung der Luft in den jeweiligen Raum eine gewisse Absenkung der Temperatur, bevorzugt um 1°C bis 7°C gegenüber der sonst im Raume herrschenden Temperatur, z.B. um etwa 4°C bis 6°C, erreicht werden kann, um so für Schädlinge ein ungünstigeres Klima zu schaffen. Zur Regelung kann die Klimaanlage dabei an mindestens einen Temperatursensor 13a angeschlossen sein.

Hiezu kann an sich jede beliebige Anordnung gewählt werden, beispielsweise ein von Kühlmittel (z.B. ein verdampfbares Kühlmittel, gegebenenfalls auch nur Kühlwasser) durchströmter Wärmetauscher und/oder bevorzugt ein Kälteerzeuger, sei es nach dem Absorber-, sei es nach dem Kompressorprinzip. Die Aufstellung des Kälteerzeugers erfolgt zweckmässig mit dem Kompressor und dem Kondensator ausserhalb der betreffenden Betriebsräumlichkeiten.

In Fig. 2 ist ein Rohrquerschnitt der Leitung 1a dargestellt. Dieser Querschnitt hat hier eine ovale Form, kann aber an sich jede beliebige zweckmässige Form annehmen. Innerhalb des Querschnittes ist ein Staubkonzentrationsmesser 2 (vgl. Fig. 1) als eine Art Lichtschranke mit einem Sender 17, z.B. auch einem Laser, wie einer Laserdiode, und einem Empfänger 18 angeordnet, die nach Art eines Trübungsmessers, auf Grund der Blendenwirkung des durch das Rohr 1a hindurchgeführten Staubes, die Staubkonzentration misst. Es mag zweckmässig sein, aus dem Strahlengang des Senders 17 unmittelbar vor demselben einen Teil des ausgesandten Lichtes abzuzweigen und einem weiteren, nicht dargestellten Empfänger zuzuführen, der als Referenzempfänger dient, da das auf ihn fallende Licht (dieser Ausdruck soll auch elektromagnetische Strahlung umfassen, die dem sichtbaren Bereiche nur benachbart ist, wie z.B. Infrarot) vom Staub nicht getrübt ist. Auf diese Weise kann das Ausgangssignal dieses zusätzlichen, nicht dargestellten Empfängers mit dem des Empfängers 18, z.B. in einer die beiden Empfänger enthaltenden Brückenschaltung, verglichen werden, um so Veränderungen der Leistung des Senders 17, bspw. durch Verschmutzung, aus dem Messergebnis auszuschalten.

Ferner wäre es denkbar, einen Luftvorhang mittels über die Fläche von Sender und/oder Empfänger blasender Reinluft oder durch gegen die Fläche dieser Teile gerichtete Reinigungsblasdüsen entweder ständig, oder aber bevorzugt impulsweise, zu schaffen und damit die Flächen von Staub frei zu halten bzw. zu reinigen. Dabei kann die Reinigung gewünschtenfalls durch den genannten zusätzlichen Empfänger gesteuert werden, indem dessen Anfangssignal (bei sauberen Flächen) gespeichert (z.B. eine Sample-and-Hold-Schaltung oder ein RAM) und periodisch mit seinem augenblicklichen Signal verglichen wird, wobei die Reinigung beim Absinken unter eine vorbestimmte Schwelle (z.B. Schwellwertschalter) ausgelöst wird. Dabei ist eine vorbestimmte Hysterese dieses Steuersystems von Bedeutung, da andernfalls die Auslöserate zu hoch wird.

Obwohl der gezeigte Staubkonzentrationsmesser bevorzugt ist und sich in der Praxis auch bewährt hat, ist die Erfindung durchaus darauf nicht beschränkt, sondern umfasst auch andere Ausführungsformen, die demselben Zweck dienen. Vor allem ist es auch möglich, den in Fig. 2 gezeigten optischen Sensor als Reflexionslichtschranke auszubilden, was den Vorteil hat, dass das Licht zweimal den Staub durchschreiten muss und von ihm getrübt wird. Ein weiterer Vorteil besteht darin, dass Sender und Empfänger an einem einzigen Ort als Baueinheit angebracht werden können.

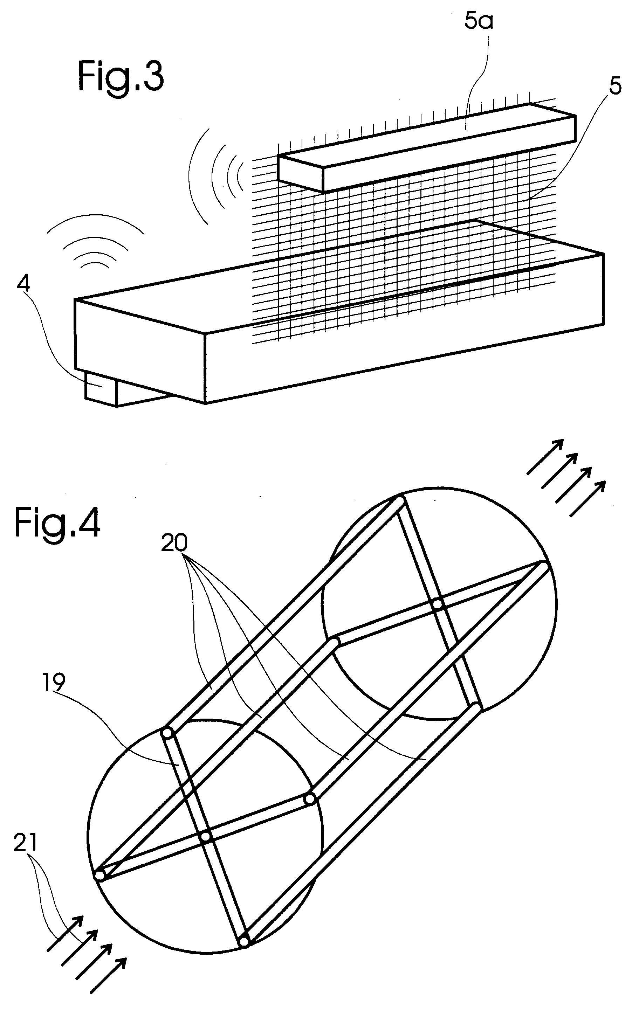

In Fig. 3 ist über den, der Übersichtlichkeit halber hier weggelassenen, beispielsweise etwa quadratischen Rohrquerschnitt, das schon erwähnte Grobsieb 5 gespannt, an dessen Oberseite die Vibrationseinrichtung 5a befestigt ist. Das Sieb 5 ist zweckmässig in einem (hier nicht dargestellten) Rahmen befestigt, der in eine Gleitschienenführung (nicht gezeigt) des Rohres von oben her einschiebbar ist. Gegebenenfalls können zwei solcher Führungen nebeneinander angeordnet sein, so dass beim Wechseln des einen Siebes 5 bereits ein anderes eingesetzt werden kann. In diesem Fall muss die zweite (leere) Führung für sich abdichtbar sein, wogegen die Abdichtung gegen das Sieb 5 durch am Siebrahmen anliegende Dichtlippen od.dgl. erfolgt.

Die Strömungsrichtung ist in Fig. 3 von links nach rechts gedacht. Daher liegt die mit dem Sieb 5 bzw. seiner (nicht dargestellten) Führung eine Einheit bildende Bestrahlungseinrichtung 4 in Strömungsrichtung vor dem Sieb 5 und sendet dort die die Parasiten tötenden Wellen aus, wie in Fig. 3 angedeutet ist. Um zu verhindern, dass Käferleichen u.dgl. mit dem Luftstrom mitgeschleppt werden, ist das Grobsieb 5 unmittelbar anschliessend vorgesehen. Wenn man bedenkt, dass die Eier von Reismehlkäfern eine Grösse von etwa 0.4 bis 0.7 mm besitzen, so ist verständlich, dass das Sieb zweckmässig eine leicht geringere Maschenweite besitzt. Beispielsweise ist eine Maschenweite von 0.3 mm vorteilhaft.

Fig. 4 veranschaulicht den Fall, dass eine UV-Bestrahlungseinrichtung an einem etwa kreisförmigen Rohrquerschnitt anzubringen ist. Entweder werden längliche UV-Röhren 20 in Schlitze der Peripherie des Rohres eingelegt, oder das Rohr wird in diesem Abschnitt aus Quarzglas hergestellt, oder - was einfacher ist - es ist im Rohrquerschnitt ein Tragstern 19 an jedem Ende der Röhren untergebracht, was den Strömungsquerschnitt kaum ernstlich verringert. Dabei sind die Röhren 20 zweckmässig jeweils in gleichmässigen Winkelabständen angeordnet, wodurch sich gegen die Rohrmitte hin eine Wirkungskonzentration ergibt.

Auch hier wird eine Leistungsregelung über die verschiedenen, oben besprochenen Sensoren 2a, E1-E3 bzw. 13a oder wenigstens einen Teil davon vorteilhaft sein, um die Leistung an vorhandene Keimzahlen anzupassen. Diese können beispielsweise periodisch an Hand von Luftproben bestimmt werden. Danach stellt man die Leistung ein, gegebenenfalls von Hand aus. Eine andere Möglichkeit besteht darin, einen Probenehmer in die Leitung 1a einzubauen, z.B. im Bereich der Ansaugung 1, und diese Proben einem automatischen Messgerät, das zweckmässig ein optisches Messgerät sein kann, zuzuführen. Im Fall eines optischen Messgerätes wäre auch an eine Bildauswertung mittels einer Vergrösserungsoptik und einer Kamera zu denken. Es versteht sich, dass die Länge der Röhren 20 leicht den besonderen Verhältnissen und der abzutötenden Keimzahl angepasst werden kann, so dass beim Hindurchströmen der Luft im Sinn der Pfeile 21 die Verweilzeit im Wirkungsbereich der UV-Strahlung entsprechend gewählt werden kann.

## Patentansprüche

1. Verfahren zum Reinigen von Luft, welche von Schädlingen bzw. Mikroorganismen metabolisierbare Stäube enthält, unter Anwendung von UV-Bestrahlung und Filtrierung der Luft, **dadurch gekennzeichnet**, dass vorgängig eine Bestrahlung mit Ultraschall und/oder Hochfrequenz durchgeführt wird, worauf in einer zweiten Stufe die UV-Bestrahlung und eine mindestens einstufige Filtrierung erfolgt.

2. Verfahren nach Anspruch 1, dadurch gekennzeichnet, dass im Anschluss an die Ultraschallbehandlung eine erste Grobsiebung zum Abscheiden abgetöteter Parasiten durchgeführt wird, vorzugsweise mit einer Maschenweite von 0.1 bis 0.4 mm, insbesondere von etwa 0.25 bis 0.3 mm.

3. Verfahren nach Anspruch 1 oder 2, dadurch gekennzeichnet, dass in der zweiten Stufe eine Ionisierung der Luft, insbesondere mit veränderbarer Leistung, durchgeführt wird.

4. Verfahren nach einem der vorhergehenden Ansprüche, dadurch gekennzeichnet, dass in der zweiten Stufe eine Desinfektionsbehandlung mit wenigstens einer der folgenden Massnahmen durchgeführt wird:
a. es wird wenigstens ein Desinfektionsmittel, vorzugsweise als Aerosol, in die Luft eingesprüht;
b. das Desinfektionsmittel ist eine quaternäre Ammoniumverbindung;
c. das Desinfektionsmittel wird vor einer allfälligen Ionisierung eingesprüht;
d. das Desinfektionsmittel wird auf wenigstens ein Filter gesprüht.

5. Verfahren nach einem der vorhergehenden Ansprüche, dadurch gekennzeichnet, dass in der zweiten Stufe zuerst wenigstens eine Filtrierung vor der UV-Bestrahlung, und gegebenenfalls vor der Ionisierung, durchgeführt wird.

6. Verfahren nach einem der vorhergehenden Ansprüche, dadurch gekennzeichnet, dass die Luft in der zweiten Stufe gekühlt wird, insbesondere erst unmittelbar vor ihrer Rückführung in den jeweiligen Raum, bevorzugt um 1°C bis 7°C gegenüber der sonst im Raum herrschenden Temperatur, z.B. um etwa 4°C bis 6°C.

7. Verfahren nach einem der vorhergehenden Ansprüche, dadurch gekennzeichnet, dass die Filtrierung in wenigstens zwei Stufen, nämlich einer Grob- und einer Feinfiltrierung, mit wenigstens einer der folgenden Massnahmen durchgeführt wird:
a. die Grobfiltrierung erfolgt für eine Korngrösse von 1 bis 1.5 µm und grösser;
b. die Feinfiltrierung erfolgt für eine Korngrösse von 1 bis 1.5 µm und kleiner;
c. die Feinfiltrierung erfolgt in einer Kaskade, insbesondere in einer Kammerfilterkaskade;
d. der Filtrierung wird eine Schalldämpfung, vorzugsweise für eine Schalldämpfung auf maximal 60 db, insbesondere maximal 50 db zugeordnet.

8. Vorrichtung zur Durchführung des Verfahrens nach einem der vorhergehenden Ansprüche, mit einer Filtereinrichtung und einer UV-Bestrahlungseinrichtung, dadurch gekennzeichnet, dass den genannten beiden Einrichtungen eine Bestrahlungseinrichtung mit Ultraschall und/oder Hochfrequenz vorgeschaltet ist.

9. Vorrichtung nach Anspruch 8, dadurch gekennzeichnet, dass eine Ionisiereinrichtung, insbesondere mit veränderbarer Leistung, der Bestrahlungseinrichtung nachgeschaltet und/oder wenigstens ein Filter der UV-Bestrahlungseinrichtung, gegebenenfalls auch der Ionisiereinrichtung, vorgeschaltet ist.

10. Vorrichtung nach Anspruch 8 oder 9, dadurch gekennzeichnet, dass eine mit einem Vorratsbehälter für ein Desinfektionsmittel verbundene Sprüheinrichtung vorgesehen ist, die insbesondere auf wenigstens ein Filter gerichtet ist.

11. Vorrichtung nach einem der Ansprüche 8 bis 10, dadurch gekennzeichnet, dass wenigstens eines der folgenden Merkmale vorgesehen ist:
a. es sind wenigstens zwei Filter angeordnet, insbesondere für unterschiedliche Korngrössen als Grobfilter, z.B. in Form eines Metallsiebes, und als Feinfilter, wie für eine Korngrösse für 1 bis 1.5 µm und grösser einerseits und für eine Korngrösse für 1 bis 1.5 µm und kleiner andererseits;
b. für wenigstens ein Filter, insbesondere für die Feinfiltrierung, ist ein Schnellwechselsystem vorgesehen, insbesondere mit wenigstens einer austauschbaren Filterkassette und/oder eine Kaskade, insbesondere eine Kammerfilterkaskade;
c. wenigstens einem Filter ist eine Schalldämpfungseinrichtung zugeordnet, die vorzugsweise für eine Schallreduktion auf maximal 60 dB, insbesondere maximal 55 dB, ausgelegt ist.

12. Vorrichtung nach einem der Ansprüche 8 bis 11, dadurch gekennzeichnet, dass ausserdem eine Kühleinrichtung, insbesondere dem Auslasse in den Raum unmittelbar vorgeordnet, vorgesehen ist, die vorzugsweise einen mit einem Kühlmittelvorrat verbundenen wärmetauscher und/oder eine einen Verdampfer und einen Kondensator aufweisende Kühleinrichtung, insbesondere mit einem Kompressor und/oder einem nachgeschalteten Tropfenabscheider, umfasst.

13. Vorrichtung nach einem der Ansprüche 8 bis 12, dadurch gekennzeichnet, dass der Bestrahlungseinrichtung (4) für Ultraschall bzw. Hochfrequenz ein Sieb (5) zum Absieben abgetöteter Parasiten, zweckmässig unmittelbar, nachgeschaltet ist, das bevorzugt eine Maschenweite von 0.1 bis 0.4 mm, insbesondere von 0.25 bis 0.3 mm, aufweist.

14. Vorrichtung nach Anspruch 13, dadurch gekennzeichnet, dass das Sieb (5) in einer vertikalen Ebene angeordnet und vorzugsweise mit einer Vibrationseinrichtung, z.B. mit einer Frequenz unter 100 Hz, insbesondere 40-60 Hz, verbunden ist.

15. Vorrichtung nach einem der Ansprüche 8 bis 14, dadurch gekennzeichnet, dass wenigstens einer der Stufen eine Reguliereinrichtung, insbesondere eine Programmsteuereinrichtung, gegebenenfalls mit wenigstens einem die Staubbelastung anzeigenden Sensor, wie einem die Staubkonzentration erfassenden optischen Sensor, zugeordnet ist.

16. Vorrichtung nach Anspruch 15, dadurch gekennzeichnet, dass die Programmsteuereinrichtung wenigstens der Ionisiereinrichtung zur Veränderung ihrer Leistung und/ oder der Sprüheinrichtung für das Desinfektionsmittel zur Veränderung von dessen Menge über die Zeit, insbesondere zur Bestimmung von Ein- und Ausschaltzeiten, zugeordnet ist.

## Claims

1. Process for purifying air which contains dusts metabolizable by pests and/or microorganisms, with the use of UV irradiation and filtration of the air, characterized in that exposure to ultrasonics and/or high frequency is effected beforehand, after which, in a second stage, the UV irradiation and at least one-stage filtration are carried out.

2. Process according to Claim 1, characterized in that an initial coarse sieving for separating off killed parasites is carried out after the ultrasonic treatment, preferably using a mesh size of from 0.1 to 0.4 mm, in particular from about 0.25 to 0.3 mm.

3. Process according to Claim 1 or 2, characterized in that ionization of the air, in particular with variable power, is carried out in the second stage.

4. Process according to any of the preceding Claims, characterized in that a disinfection treatment with at least one of the following measures is carried out in the second stage:
a. at least one disinfectant, preferably an aerosol, is sprayed into the air;
b. the disinfectant is a quaternary ammonium compound;
c. the disinfectant is sprayed in prior to any ionization;
d. the disinfectant is sprayed onto at least one filter.

5. Process according to any of the preceding Claims, characterized in that, in the second stage, at least one filtration is first carried out before the UV irradiation and optionally before the ionization.

6. Process according to any of the preceding Claims, characterized in that the air is cooled in the second stage, in particular immediately before it is recycled to the respective space, preferably by 1°C to 7°C relative to the temperature otherwise prevailing in the room, for example by about 4°C to 6°C.

7. Process according to any of the preceding Claims, characterized in that the filtration is carried out in at least two stages, namely a coarse and a fine filtration, with at least one of the following measures:
a. the coarse filtration is carried out for a particle size of from 1 to 1.5 µm or larger;
b. the fine filtration is carried out for a particle size of from 1 to 1.5 µm or smaller;
c. the fine filtration is carried out in a cascade, in particular in a chamber filter cascade;
d. sound absorption, preferably for a sound absorption to a maximum level of 60 dB, in particular a maximum level of 50 dB, is coordinated with the filtration.

8. Device for carrying out the process according to any of the preceding Claims, comprising a filter means and a UV irradiation means, characterized in that said two means follow an ultrasonic and/or high frequency exposure means.

9. Device according to Claim 8, characterized in that an ionization means, in particular with variable power, is present downstream of the exposure means and/or at least one filter is present upstream of the UV irradiation means, optionally also of the ionization means.

10. Device according to Claim 8 or 9, characterized in that a spray means connected to a storage container for disinfectant is provided, which spray means is directed in particular towards at least one filter.

11. Device according to any of Claims 8 to 10, characterized in that at least one of the following features is provided:
a. at least two filters are provided, in particular for different particle sizes, as a coarse filter, for example in the form of a metal sieve, and as a fine filter, for a particle size of from 1 to 1.5 µm or larger on the one hand and for a particle size of from 1 to 1.5 µm or smaller on the other hand;
b. a fast-change system, in particular having at least one replaceable filter cassette and/or a cascade, in particular a chamber filter cascade, is provided for at least one filter, in particular for the fine filtration;
c. a sound absorption means which is preferably designed for a sound reduction to a maximum level of 60 dB, in particular to a maximum level of 55 dB, is coordinated with at least one filter.

12. Device according to any of Claims 8 to 11, characterized in that furthermore a cooling means is provided, in particular arranged directly before the outlet into the space, which cooling means preferably comprises a heat exchanger connected to a coolant reservoir and/or a cooling apparatus having an evaporator and a condenser, in particular having a compressor and/or a downstream drop separator.

13. Device according to any of Claims 8 to 12, characterized in that a sieve (5) for separating off killed parasites is present downstream, expediently directly downstream, of the exposure means (4) for ultrasonics and/or high frequency, said sieve preferably having a mesh size of from 0.1 to 0.4 mm, in particular from 0.25 to 0.3 mm.

14. Device according to Claim 13, characterized in that the sieve (5) is arranged in a vertical plane and is preferably connected to a vibration means, for example with a frequency of less than 100 Hz, in particular 40-60 Hz.

15. Device according to any of Claims 8 to 14, characterized in that a regulation means, in particular a program control means, optionally having at least one sensor indicating the dust contamination, such as an optical sensor determining the dust concentration, is coordinated with at least one of the stages.

16. Device according to Claim 15, characterized in that the program control means is coordinated with at least the ionization means for changing its power and/or the spray means for the disinfectant in order to change the amount thereof as a function of time, in particular for determining the switching on and switching off times.

## Revendications

1. Procédé de purification de l'air, contenant des poussières susceptibles d'être métabolisées par des parasites, respectivement des micro-organismes, avec une utilisation d'une irradiation aux UV et filtration de l'air, caractérisé en ce que, préalablement, on effectue une irradiation aux ultrasons et/ou à haute fréquence, à la suite de quoi, dans une deuxième étape, on effectue l'irradiation aux UV et une filtration, faite au moins en une étape.

2. Procédé selon la revendication 1, caractérisé en ce que, suite au traitement aux ultrasons, on effectue un tamisage grossier en vue de séparer les parasites tués, de préférence en utilisant une largeur de mailles de 0,1 à 0,4 mm, en particulier d'environ 0,25 à 0,3 mm.

3. Procédé selon la revendication 1 ou 2, caractérisé en ce que, dans la deuxième étape, on effectue une ionisation de l'air, en particulier avec une puissance susceptible d'être modifiée.

4. Procédé selon l'une des revendications précédentes, caractérisé en ce que, dans la deuxième étape, on effectue un traitement de désinfection à l'aide d'au moins l'une des dispositions ci-après :
a. on injecte dans l'air au moins un agent désinfectant, de préférence sous forme d'aérosol;
b. l'agent désinfectant est une combinaison ammonium quaternaire;
c. l'agent désinfectant est injecté avant une éventuelle ionisation;
d. l'agent désinfectant est pulvérisé sur au moins un filtre.

5. Procédé selon l'une des revendications précédentes, caractérisé en ce que, dans la deuxième étape, on effectue d'abord au moins une filtration avant l'irradiation UV et, le cas échéant, avant l'ionisation.

6. Procédé selon l'une des revendications précédentes, caractérisé en ce que l'air est refroidi dans la deuxième étape, en particulier ensuite, directement avant son recyclage dans le local respectif, de préférence d'une température de 1 °C a 7 °C par rapport à la température régnant par ailleurs dans le local, par exemple est refroidi d'environ 4 °C à 6 °C.

7. Procédé selon l'une des revendications précédentes, caractérisé en ce que la filtration est effectuée en au moins deux étapes, précisément une filtration grossière et une filtration fine, avec au moins l'une des dispositions ci-après :
a. la filtration grossière s'effectue pour une taille de grains de 1 a 1,5 µm et plus;
b. la filtration fine s'effectue pour une taille de grains de 1 a 1,5 µm et moins;
c. la filtration fine s'effectue en cascade, en particulier dans une cascade de filtres a chambres;
d. à la filtration est associée une atténuation sonore, de préférence permettant d'obtenir une atténuation sonore jusqu'à un maximum de 60 dB, en particulier un maximum de 50 dB.

8. Dispositif de mise en oeuvre du procédé selon l'une des revendications précédentes avec un dispositif de filtration et un dispositif d'irradiation aux UV, caractérisé en ce qu'on met en circuit en amont des deux dispositifs cités un dispositif d'irradiation aux ultrasons et/ou à haute fréquence.

9. Dispositif selon la revendication 8, caractérisé en ce qu'un dispositif d'ionisation, en particulier ayant une puissance pouvant être modifiée, est mis en circuit en aval du dispositif d'irradiation et/ou au moins un filtre est mis en circuit en amont du dispositif d'irradiation aux UV, le cas échéant également en amont du dispositif d'ionisation.

10. Dispositif selon la revendication 8 ou 9, caractérisé en ce qu'est prévu un dispositif de pulvérisation relié a un récipient de stockage pour un agent désinfectant, dispositif de pulvérisation dirigé en particulier sur au moins un filtre.

11. Dispositif selon l'une des revendications 8 a 10, caractérisé en ce qu'est prévue au moins l'une des caractéristiques ci-après :
a. il est disposé au moins deux filtres, en particulier pour des tailles de grains différentes, a titre de filtre grossier, par exemple sous la forme d'un tamis métallique, et sous la forme d'un filtre fin comme pour une taille de grains comprise dans la plage allant de 1 à 1,5 µm et plus, d'une part, et pour une taille de grains comprise dans la plage allant de 1 a 1,5 µm et moins, d'autre part;
b. pour au moins un filtre, en particulier pour le filtrage fin, est prévu un système de remplacement rapide, en particulier faisant appel a au moins une cassette de filtre interchangeable et/ou une cascade, en particulier une cascade de filtres a chambres;
c. à au moins un filtre est associé un dispositif d'atténuation sonore qui est conçu de préférence pour obtenir une réduction du bruit jusqu'à un maximum de 60 dB, en particulier un maximum de 55 dB.

12. Dispositif selon l'une des revendications 8 à 11, caractérisé en ce qu'en outre est prévu un dispositif de refroidissement, disposé en particulier directement devant les évacuations, dans le local, dispositif de refroidissement qui comprend de préférence un échangeur de chaleur relié a un stockage d'agent de refroidissement et/ou un dispositif de refroidissement présentant un évaporateur et un condenseur, en particulier avec un compresseur et/ou un séparateur à gouttelettes, mis en circuit en aval.

13. Dispositif selon l'une des revendications 8 à 12, caractérisé en ce qu'en aval du dispositif d'irradiation (4) destiné aux ultrasons ou aux hautes fréquences est disposé, de manière appropriée, directement un tamis (5) destiné à la séparation par tamisage des parasites tués, qui, de préférence, présente une largeur de mailles comprise dans la plage allant de 0,1 à 0,4 mm, en particulier de 0,25 à 0,3 mm.

14. Dispositif selon la revendication 13, caractérisé en ce que le tamis (5) est disposé dans un plan vertical et, de préférence, est relié à un dispositif vibreur, par exemple travaillant à une fréquence inférieure a 100 Hz, en particulier une fréquence comprise dans la plage allant de 40 à 60 Hz.

15. Dispositif selon l'une des revendications 8 à 14, caractérisé en ce que, au moins à l'une des étapes est associé un dispositif de régulation, en particulier un dispositif de commande programmé, le cas échéant avec un capteur affichant la teneur en poussières, tel qu'un capteur optique appréhendant la concentration en poussières. 16. Dispositif selon la revendication 15, caractérisé en ce que le dispositif de commande programmé est associé au moins au dispositif d'ionisation, en vue de modifier sa puissance, et/ou au dispositif de pulvérisation destiné a l'agent désinfectant, en vue de modifier son débit en fonction du temps, en particulier pour déterminer les temps de mise en service et hors service.
